(19) Europäisches Patentamt
European Patent Office
Office européen des brevets

(11) **EP 2 017 623 B1**

(12) **FASCICULE DE BREVET EUROPEEN**

(45) Date de publication et mention
de la délivrance du brevet:
**11.08.2010 Bulletin 2010/32**

(51) Int Cl.:
***G01N 33/68*** *(2006.01)* ***G01N 33/84*** *(2006.01)*

(21) Numéro de dépôt: **08011900.1**

(22) Date de dépôt: **02.07.2008**

(54) **Procédé de prédiction d'un score représentatif du stress oxydatif d'un patient**

Verfahren zur Vorhersage eines repräsentativen Scores des oxidativen Stresses eines Patienten

Method of predicting a score representing a patient's oxidative stress

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **16.07.2007 FR 0705105**

(43) Date de publication de la demande:
**21.01.2009 Bulletin 2009/04**

(73) Titulaire: **Swiss Line Services Ltd.**
**Central**
**Hong Kong (CN)**

(72) Inventeurs:
• **Brack, Michel**
**92700 Colombes (FR)**
• **Brack, Olivier**
**60110 Esches (FR)**

(74) Mandataire: **Micheli & Cie SA**
**Rue de Genève 122**
**Case Postale 61**
**1226 Genève-Thônex (CH)**

(56) Documents cités:
**EP-A- 1 793 230**

• **SCHISTERMAN E F ET AL: "Minimal and best linear combination of oxidative stress and antioxidant biomarkers to discriminate cardiovascular disease." NUTRITION, METABOLISM, AND CARDIOVASCULAR DISEASES : NMCD OCT 2002, vol. 12, no. 5, octobre 2002 (2002-10), pages 259-266, XP008085094 ISSN: 0939-4753**
• **BEN-DOR A ET AL ASSOCIATION FOR COMPUTING MACHINERY: "TISSUE CLASSIFICATION WITH GENE EXPRESSION PROFILES" RECOMB 2000. PROCEEDINGS OF THE 4TH. ANNUAL INTERNATIONAL CONFERENCE ON COMPUTATIONAL MOLECULAR BIOLOGY. TOKYO, JAPAN, APRIL 8 - 11, 2000, PROCEEDINGS OF THE ANNUAL INTERNATIONAL CONFERENCE ON COMPUTATIONAL MOLECULAR BIOLOGY, NEW YORK, NY : ACM, US, vol. CONF.4, 8 avril 2000 (2000-04-08), pages 54-64, XP001049478 ISBN: 1-58113-186-0**
• **MAMTANI MANJU R ET AL: "A simple method to combine multiple molecular biomarkers for dichotomous diagnostic classification." BMC BIOINFORMATICS 2006, vol. 7, 2006, page 442, XP002456260 ISSN: 1471-2105**
• **VEGLIA F ET AL: "Age- and gender-related oxidative status determined in healthy subjects by means of OXY-SCORE, a potential new comprehensive index" BIOMARKERS, vol. 11, no. 6, novembre 2006 (2006-11), pages 562-573, XP008085093 ISSN: 1354-750X**

**Description**

La présente invention a pour objet un procédé de prédiction d'un score représentatif du stress oxydatif d'un patient sur une échelle de 0 à 10.

Il est connu que l'oxygène est le carburant des cellules, et il existe dans la littérature de nombreuses publications à ce sujet.

Toutefois, certaines formes d'oxygène, notamment les radicaux libres, peuvent être fortement toxiques pour l'organisme et les cellules dans la mesure où elles catalysent des réactions d'oxydation de nature à altérer au hasard les molécules biologiques.

Il s'agit là du phénomène connu des spécialistes sous le nom de stress oxydatif.

Ce phénomène est impliqué dans une large gamme de pathologies, en particulier les hépatites, le cancer, le sida, la maladie d'Alzheimer, la maladie de Parkinson... et correspond en outre au moteur essentiel du vieillissement.

Or, le corps médical dispose aujourd'hui de moyens permettant d'intervenir sur le stress oxydatif tels qu'à titre d'exemple une amélioration de l'hygiène de vie, une prise en charge nutritionnelle (suivant les recommandations du Programme National Nutrition Santé) ou encore l'administration d'antioxydants naturels ou de synthèse.

Il est donc souhaitable de pouvoir évaluer le stress oxydatif dans chaque cas particulier afin de prédire un risque de développement d'une pathologie et de prendre précocement, dès la détection d'une anomalie, toutes les mesures possibles pour y remédier.

A cet effet, différents laboratoires proposent actuellement d'explorer ce stress à partir de bilans essentiellement sanguins basés sur le dosage de certains marqueurs biologiques.

Il s'agit toutefois là de démarches largement empiriques, ce d'autant plus que les marqueurs utilisés varient largement d'un laboratoire à un autre.

En conséquence, de tels bilans ne sont pas de nature à permettre une prise en charge du stress oxydatif en médecine courante et leur interprétation qui est particulièrement complexe est l'apanage de quelques spécialistes rodés au stress oxydatif depuis plusieurs années.

Plus récemment, et selon le document EP 1 793 230, on a proposé de déterminer à partir de bilans sanguins et de dosages biologiques un score représentatif du stress oxydatif d'un patient, sur une échelle de 0 à 10 (du bilan le moins perturbé au bilan le plus perturbé).

Un tel score constitue un outil scientifique offert au corps médical pour effectuer une évaluation du stress oxydatif d'un patient dans la mesure où il est indicatif d'un bilan normal ou plus ou moins anormal.

En présence d'un bilan anormal, le médecin doit ensuite faire un travail d'interprétation pour évaluer l'origine de cette anomalie et prescrire au patient les mesures nécessaires pour y remédier.

La pertinence de ces mesures (conseils sur l'hygiène de vie, modification du régime alimentaire, en particulier par augmentation de l'apport en fruits et légumes ou encore supplémentation antioxydante) peut être contrôlée après quelques mois grâce à une nouvelle détermination du score représentatif du stress oxydatif.

Un tel procédé de détermination d'un score représentatif du stress oxydatif d'un patient présente toutefois l'inconvénient de nécessiter la prise en compte d'une quinzaine de marqueurs biologiques regroupés en trois catégories, exprimant respectivement des informations différentes pouvant être associées à des stades différents de développement du stress oxydatif et correspondant respectivement à des antioxydants exogènes à des antioxydants endogènes et à des marqueurs de dégâts oxydatifs. Par suite ce procédé est associé à un coût relativement élevé.

Il serait par suite souhaitable de mettre en oeuvre avant la détermination de ce score une étape de « sondage » préalable permettant de prédire la plage dans laquelle il se situera en ne prenant en compte qu'un nombre restreint de marqueurs biologiques présélectionnés de façon à obtenir à moindre coût une prédiction suffisamment précise et fiable.

Un tel « sondage » pourrait en effet permettre d'établir un classement préalable des patients en fonction de leur risque de stress oxydatif pour ne soumettre à un bilan complet que les patients pour lesquels un risque de stress oxydatif élevé a été auparavant déterminé.

La présente invention a pour objet de proposer un procédé de prédiction d'un score représentatif du stress oxydatif d'un patient sur une échelle de 0 à 10 de nature à satisfaire à cette demande.

Selon l'invention, ce procédé est caractérisé par la succession des étapes suivantes :

- on sélectionne un parmi les deux groupes de cinq marqueurs biologiques suivants :

  - le premier groupe étant constitué: du cuivre Cu, du zinc Zn, du rapport glutathion réduit/glutathion oxydé (ratio G/G), des protéines thiols (prot th) et du malon dialdéhyde plasmatique (MDA) et ;
  - le second groupe étant constitué : du cuivre Cu, du zinc Zn, des protéines thiols (prot. th), du malon dialdéhyde plasmatique (MDA) et des T-Bars, c'est-à-dire des substances qui réagissent avec l'acide thio-barbiturique ;

- on évalue par dosage, en particulier à partir de prélèvements sanguins, les marqueurs biologiques du groupe sélectionné du patient, et

- on calcule le score prédictif Sp spécifique au patient en utilisant une relation de prédiction, basée sur le dosage des cinq marqueurs du groupe sélectionné.

Le procédé conforme à l'invention a pu être conçu à l'origine, grâce à un travail pluridisciplinaire, médical et mathématique.

On a ainsi préalablement établi une série de bilans de stress oxydatif complets conformément au document EP 1 793 230 et on a fait valider chacun de ces bilans par un expert médical pour éliminer les bilans atypiques.

On a ainsi obtenu à partir de 177 bilans de stress oxydatif une base de données très complète qui figure en annexe 1 à titre d'exemple.

Les significations des abréviations des différents marqueurs biologiques utilisés, leurs unités et les normes sont précisées en annexe 2.

On a ensuite effectué un traitement statistique de cette base de données de façon à sélectionner les marqueurs les plus pertinents d'un point de vue médical et les plus aptes à prédire le score de stress oxydatif.

Selon l'invention, le groupe de marqueurs biologiques ainsi sélectionnés peut avantageusement renfermer moins de 6 marqueurs biologiques.

Selon une première variante de l'invention, ces marqueurs biologiques sont le cuivre Cu, le zinc Zn, le rapport glutathion réduit/glutathion oxydé(ratio G/G), les protéines thiols (prot th) et le malon dialdéhyde plasmatique (MDA).

Selon cette variante de l'invention, il est ainsi proposé d'obtenir une prédiction suffisamment précise et fiable du score oxydatif d'un patient à partir de seulement cinq marqueurs biologiques judicieusement sélectionnés (nécessitant six dosages).

Il est à noter que ces marqueurs biologiques appartiennent aux trois catégories de marqueurs biologiques prises en considération dans le cadre du procédé de détermination du stress oxydatif susmentionné, à savoir des marqueurs exogènes (Cu, Zn) des marqueurs endogènes (ratio G/G, prot th) et des marqueurs de dégâts oxydatifs (MDA).

Le traitement statistique effectué a ensuite consisté à rechercher, à partir des marqueurs biologiques ainsi sélectionnés, la relation de prédiction la mieux adaptée pour prédire avec suffisamment de précision quel sera le score de stress oxydatif d'un patient ou en d'autres termes pour obtenir un score prédictif Sp le plus proche possible du score de stress oxydatif S0.

Selon la première variante de l'invention, la relation de prédiction ainsi déterminée est la relation suivante :

$$Sp = 6.8127106 + 0.5105519 \times Cu/Zn - 0.026794 \times Ratio\ G/G - 0.671527 \times prot\ th + 1.5006322 \times MDA + 0.0001327 \times (ratio\ G/G-63.8422) \times (ratio\ G/G-63.8422) - 0.623101 \times (MDA-2.03113) \times (MDA-2.03113)$$

Selon cette première variante de l'invention il est proposé de classer les scores prédictifs en trois classes de risques.

Selon la classe dans laquelle est situé le score prédictif d'un patient, le corps médical peut prendre la décision la plus appropriée pour son suivi, à savoir :

- classe 1 : note inférieure à 4,5,
  aucun suivi spécifique du patient,
- classe 2 : note comprise entre 4,5 et 5,5,
  risque moyen ; les patients se trouvant dans cette zone peuvent subir un traitement pendant trois à six mois avant de subir une nouvelle épreuve pour déterminer si leur stress oxydatif a ou non diminué,
- classe 3 : note supérieure à 5,5
  risque élevé ; les patients se trouvant dans cette zone doivent subir un bilan complet de stress oxydatif ou d'autres explorations.

Selon une seconde variante de l'invention les marqueurs biologiques sélectionnés sont le cuivre Cu, le zinc Zn, les protéines thiols (prot th) le ma-lon dialdéhyde plasmatique (MDA) et les T-Bars, c'est-à-dire les substances qui réagissent avec l'acide thio-barbiturique.

Il est à noter que les T-Bars correspondent à un marqueur de type 3 (dégâts oxydatifs majeurs).

Selon cette seconde variante, en vue d'éviter le dosage du Ratio G/G, nécessitant des précautions particulières lors du prélèvement et principalement lors du stockage des échantillons (congélation à -80°C), il a été décidé d'évaluer un modèle alternatif en l'absence de ce dosage.

Le traitement statistique effectué a conduit à déterminer le score prédictif Sp' à partir de la relation de prédiction

suivante

$$Sp' = 3.0818 + 0.27018 \times Cu/Zn - 0.64301 \times prot\ th + 0.91474 \times MDA$$
$$+ 1.16677 \times T\text{-}Bars + 0.99643 \times (Cu/Zn - 1.52488) \times (MDA - 2.03113) + 0.9099 \times$$

$$(Cu/Zn - 1.52488) \times (T\text{-}Bars - 2.91639) + 0.29203 \times (prot\ th - 5.9951) \times (MDA - 2.03113)$$

Il est à noter que selon cette seconde variante de l'invention les classes de score prédictif sont un peu décalées par rapport aux classes définies selon la première variante ; en effet :

- la classe 1 correspond à une note inférieure à 4,
- la classe 2 correspond à une note comprise entre 4 et 6,
- la classe 3 correspond à une note supérieure à 6.

Pour vérifier la représentativité du score prédictif du stress oxydatif obtenu par la mise en oeuvre du procédé conforme à l'invention on a calculé chez un panel de patients :

- d'une part le score de stress oxydatif conformément au document EP 1 793 230, et
- d'autre part le score prédictif conformément aux deux variantes de l'invention.

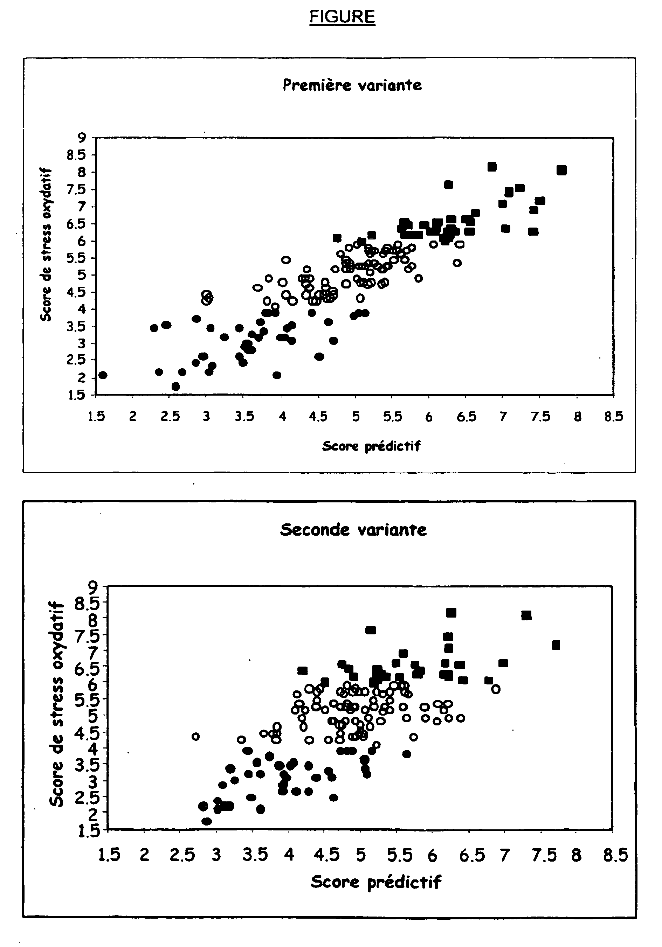

Les résultats obtenus sont rassemblés sur la figure annexée sur laquelle le score prédictif est porté en abscisse alors que le score de stress oxydatif est porté en ordonnée.
Les cercles noirs correspondent à des patients appartenant à la classe 1 tandis que les cercles clairs correspondent à des patients appartenant à la classe 2 et les carrés noirs à des patients appartenant à la classe 3.
Ces résultats sont clairement de nature à prouver la qualité des scores prédictifs obtenus conformément aux deux variantes de l'invention.
Il est à noter que d'un point de vue purement statistique, ces travaux ont fait appel aux techniques et méthodes suivantes :

- Analyses en Composantes Principales,
- Régressions Linéaires Multiples (moindres carrés) et Régressions PLS (Partial Least Squares),
- Classification Hiérarchique et Analyse de Tableaux de Contingence (Test du Khi$^2$ et Indice Kappa).

Il est à noter que la prédiction du score représentatif du stress oxydatif d'un patient S0 et l'affectation à la classe de risque associée sont actuellement réalisables de façon interactive grâce à un utilitaire développé sous Microsoft® Exel dans sa version 2003 professionnelle sous la forme d'une macro commande.
Pour l'ensemble des traitements statistiques, le logiciel Jmp dans sa version 6.0.2 (Statical Discovery TM from SAS - Copyright© 2006 SAS Institute Inc) a été utilisé.

ANNEXE 1

Annexe I - Base de données

| Patient | Vit C | Alpha Toco | Gamma tocoph | Vit C/E | GSH | GSSG | Ratio G/G | GPX | Prot Thiol | Acide Urique | Sel | Cu | Zn | Cu/Zn | MDA | Tbars | Oxyscale |
|---|---|---|---|---|---|---|---|---|---|---|---|---|---|---|---|---|---|
| Patient 1 | 54 | 22.5 | 1.71 | 2.400 | 771 | 23.4 | 32.95 | 416 | 6.41 | 190.4 | 1.14 | 15.80 | 9.4 | 1.680 | 3.11 | 3.7 | 6.64 |
| Patient 2 | 60 | 23 | 1.45 | 2.610 | 1300 | 34 | 38.24 | 567 | 4.5 | 300 | 1.34 | 20.00 | 16 | 1.250 | 1 | 2 | 4.73 |
| Patient 3 | 60 | 21.5 | 1.72 | 2.791 | 1449 | 23 | 63.00 | 385 | 5.67 | 531 | 1.32 | 17.00 | 11.2 | 1.518 | 3.04 | 2.81 | 6.27 |
| Patient 4 | 79 | 26.4 | 1.37 | 2.992 | 915 | 7.5 | 122.00 | 377 | 3.84 | 260 | 1.33 | 19.50 | 10.5 | 1.857 | 2.18 | 3.17 | 4.91 |
| Patient 5 | 86.4 | 46.8 | 3.12 | 1.850 | 792 | 4 | 198.00 | 521 | 5.5 | 245 | 1.75 | 26.60 | 9.8 | 2.710 | 2.25 | 3 | 5.18 |
| Patient 6 | 32.2 | 23 | 1.83 | 1.400 | 723 | 8.5 | 85.06 | 452 | 5.51 | 333.2 | 1.09 | 16.80 | 11 | 1.530 | 2.01 | 2.75 | 4.55 |
| Patient 7 | 44 | 25 | 0.85 | 1.760 | 652 | 14.7 | 44.35 | 344 | 5.97 | 208.2 | 1.12 | 18.00 | 14.1 | 1.280 | 1.96 | 249 | 5.73 |
| Patient 8 | 93.2 | 31.4 | 2.02 | 2.968 | 790 | 5 | 158.00 | 395 | 5.8 | 205 | 1.36 | 19.20 | 11.2 | 1.714 | 2.46 | 3.44 | 4.91 |
| Patient 9 | 70.3 | 33.8 | 1.3 | 2.080 | 603 | 12 | 50.25 | 430 | 6.8 | 284 | 1.09 | 18.60 | 12.4 | 1.500 | 1.86 | 2.19 | 4.27 |
| Patient 10 | 81 | 27.3 | 2.04 | 2.970 | 1054 | 22.5 | 46.84 | 363 | 7.1 | 338 | 1.11 | 13.40 | 14 | 0.960 | 1.36 | 2.08 | 2.36 |
| Patient 11 | 54 | 29.1 | 2.17 | 1.860 | 887 | 15.9 | 55.79 | 407 | 5.97 | 169 | 1.42 | 19.20 | 8.5 | 2.260 | 1.78 | 3.19 | 6.00 |
| Patient 12 | 121.4 | 28.4 | 0.5 | 4.270 | 896 | 22 | 41.67 | 429 | 6.33 | 184.4 | 1.5 | 19.40 | 11.80 | 1.640 | 2 | 3.07 | 5.27 |
| Patient 13 | 22.5 | 26.4 | 1.32 | 0.850 | 714 | 11.4 | 62.63 | 448 | 5.46 | 416.5 | 0.75 | 14.60 | 14.8 | 0.990 | 1.86 | 3.18 | 6.09 |
| Patient 14 | 61.4 | 20.2 | 0.86 | 3.040 | 1090 | 7.5 | 145.33 | 372 | 7.39 | 211 | 112 | 18.40 | 11.7 | 1.573 | 1.44 | 2.78 | 2.09 |

(suite)

| Patient | Vit C | Alpha Toco | Gamma tocoph | Vit C/E | GSH | GSSG | Ratio G/G | GPX | Prot Thiol | Acide Urique | Sel | Cu | Zn | Cu/Zn | MDA | Tbars | Oxyscale |
|---|---|---|---|---|---|---|---|---|---|---|---|---|---|---|---|---|---|
| Patient 15 | 87 | 25.5 | 0.84 | 3.410 | 1025 | 19.8 | 31.77 | 433 | 7 | 205 | 0.97 | 15.40 | 13.2 | 1.170 | 1.69 | 2.87 | 4.27 |
| Patient 16 | 59.4 | 352 | 1.57 | 1.690 | 842 | 12 | 70.17 | 367 | 6.52 | 351 | 1.67 | 16.20 | 13.5 | 1.200 | 2.75 | 3.65 | 3.82 |
| Patient 17 | 59 | 30.8 | 2.43 | 1.916 | 714 | 18 | 39.67 | 472 | 6.06 | 281 | 1.39 | 20.80 | 10.5 | 1.981 | 2.41 | 3.11 | 6.36 |
| Patient 18 | 48 | 24.6 | 1.76 | 1.951 | 1108 | 21 | 52.76 | 486 | 6.74 | 346 | 1.5 | 15.50 | 9.8 | 1.582 | 2.12 | 2.8 | 5.45 |
| Patient 19 | 61.4 | 21.5 | 1.22 | 2.856 | 900 | 10 | 90.00 | 400 | 6.66 | 220 | 125 | 15.80 | 13.2 | 1.197 | 1.57 | 2.3 | 2.45 |
| Patient 20 | 78 | 21.6 | 1.26 | 3.610 | 1295 | 8.5 | 152.35 | 352 | 6.26 | 183 | 1.29 | 17.00 | 9.2 | 1.850 | 2.44 | 2.65 | 1.43 |
| Patient 21 | 79.1 | 22.4 | 0.98 | 3.530 | 1146 | 18.9 | 60.63 | 343 | 6.2 | 193 | 1.86 | 17.00 | 11.2 | 1.520 | 1.65 | 2.77 | 4.00 |
| Patient 22 | 179 | 35.7 | 0.86 | 5.010 | 1004 | 12.9 | 77.83 | 498 | 5.62 | 236 | 151 | 19.40 | 13.4 | 1.430 | 2.31 | 2.94 | 5.27 |
| Patient 23 | 57.7 | 33.2 | 2.14 | 1.710 | 959.8 | 13.6 | 70.57 | 348 | 6.5 | 222 | 0.82 | 17.80 | 11.6 | 1.530 | 1.06 | 2.31 | 2.18 |
| Patient 24 | 120.7 | 23.4 | 0.95 | 5.160 | 611 | 4.8 | 127.29 | 401 | 636 | 113.1 | 0.9 | 18.20 | 14.3 | 1.270 | 1.51 | 2.75 | 3.45 |
| Patient 25 | 43 | 28.5 | 1.28 | 1.510 | 1042 | 56.2 | 18.54 | 475 | 5.8 | 306 | 136 | 18.20 | 12.4 | 1.470 | 1.67 | 2.44 | 4.91 |
| Patient 26 | 73 | 11.7 | 2 | 6.240 | 878.2 | 12.9 | 68.08 | 433 | 5.6 | 242 | 1.28 | 20.00 | 14 | 1.430 | 2.16 | 2.54 | 5.36 |
| Patient 27 | 64 | 35.9 | 1.1 | 1.780 | 863 | 29 | 29.76 | 410 | 5.31 | 399 | 1.58 | 15.80 | 10.5 | 1.500 | 1.82 | 2.95 | 6.27 |
| Patient 28 | 86.4 | 31.3 | 2.49 | 2.760 | 830 | 20.7 | 40.10 | 454 | 6.05 | 320 | 1.51 | 19.80 | 14.2 | 1.390 | 2.16 | 2.72 | 5.73 |

EP 2 017 623 B1

(suite)

| Patient | Vit C | Alpha Toco | Gamma tocoph | Vit C/E | GSH | GSSG | Ratio G/G | GPX | Prot Thiol | Acide Urique | Sel | Cu | Zn | Cu/Zn | MDA | Tbars | Oxyscale |
|---|---|---|---|---|---|---|---|---|---|---|---|---|---|---|---|---|---|
| Patient 29 | 64.2 | 21.3 | 1.36 | 3.010 | 935 | 16.2 | 57.72 | 352 | 4.81 | 379 | 0.95 | 16.20 | 12.1 | 1.340 | 1.61 | 2.28 | 3.91 |
| Patient 30 | 64 | 33.3 | 1.82 | 1.920 | 898 | 19.2 | 46.77 | 461 | 6.4 | 359 | 1.53 | 18.20 | 13.2 | 1.380 | 2.16 | 3.09 | 5.73 |
| Patient 31 | 54.4 | 24.2 | 1.25 | 2.250 | 564 | 32.5 | 17.35 | 332 | 4.16 | 266 | 0.78 | 22.20 | 10 | 2.220 | 1.15 | 2.46 | 6.00 |
| Patient 32 | 60.4 | 31.7 | 2.66 | 1.910 | 825 | 11.7 | 70.51 | 453 | 6.76 | 352 | 1.21 | 20.60 | 12 | 1.720 | 1.36 | 2.63 | 2.18 |
| Patient 33 | 65.5 | 29.4 | 1.91 | 2.230 | 916 | 22 | 41.64 | 425 | 5.33 | 362 | 1.01 | 21.50 | 12.8 | 1.680 | 2.18 | 3.44 | 6.64 |
| Patient 34 | 60.4 | 24.7 | 1.54 | 2.450 | 938 | 17.2 | 54.53 | 406 | 5.2 | 368 | 1.01 | 17.80 | 9.6 | 1.850 | 0.96 | 2.13 | 3.00 |
| Patient 35 | 71 | 27.3 | 1.31 | 2.601 | 1093 | 8.5 | 128.59 | 426 | 5.28 | 267 | 133 | 19.50 | 11.1 | 1.757 | 2.53 | 3.15 | 5.36 |
| Patient 36 | 72.8 | 53.9 | 1.17 | 1.350 | 748 | 15 | 49.87 | 424 | 5.7 | 541 | 1.8 | 15.80 | 17 | 0.930 | 1.47 | 2.78 | 4.27 |
| Patient 37 | 84 | 40.9 | 1.71 | 2.050 | 1047 | 22.4 | 46.74 | 497 | 7.1 | 200 | 1.6 | 14.18 | 15.4 | 0.920 | 1.64 | 3.04 | 4.64 |
| Patient 38 | 98.1 | 30.7 | 1.24 | 3.200 | 950 | 12.9 | 73.64 | 449 | 6.19 | 179 | 1.63 | 17.20 | 12.2 | 1.410 | 2.74 | 3.59 | 4.82 |
| Patient 39 | 79.2 | 21.3 | 0.41 | 3.718 | 1032 | 9 | 114.67 | 435 | 6.73 | 267 | 1.39 | 15.80 | 9.2 | 1.717 | 1.57 | 2.36 | 2.18 |
| Patient 40 | 32 | 38.2 | 1.39 | 0.840 | 851 | 8.3 | 102.53 | 429 | 5.32 | 220 | 1.32 | 17.00 | 10.4 | 1.630 | 2.19 | 3.06 | 5.64 |
| Patient 41 | 64.8 | 29.4 | 2.66 | 2.200 | 1001 | 24.8 | 40.36 | 473 | 5.27 | 348 | 1.12 | 22.40 | 9.7 | 2.310 | 1.77 | 3.07 | 5.91 |
| Patient 42 | 80 | 52.4 | 1.76 | 1.530 | 899 | 20.2 | 44.50 | 515 | 6.2 | 167 | 1.35 | 20.20 | 9.9 | 2.040 | 2.29 | 2.89 | 6.45 |

EP 2 017 623 B1

(suite)

| Patient | Vit C | Alpha Toco | Gamma tocoph | Vit C/E | GSH | GSSG | Ratio G/G | GPX | Prot Thiol | Acide Urique | Sel | Cu | Zn | Cu/Zn | MDA | Tbars | Oxyscale |
|---|---|---|---|---|---|---|---|---|---|---|---|---|---|---|---|---|---|
| Patient 43 | 81 | 27.5 | 0.69 | 2.950 | 1068 | 19.5 | 54.77 | 428 | 6.2 | 199 | 1.3 | 42.80 | 11.3 | 3.790 | 2.04 | 3.34 | 6.09 |
| Patient 44 | 72.6 | 25.4 | 0.89 | 2.860 | 583 | 9 | 64.78 | 415 | 4.7 | 304 | 1.4 | 26.50 | 13.5 | 1.960 | 1.85 | 2.75 | 5.45 |
| Patient 45 | 66 | 31.9 | 1.26 | 2.070 | 687 | 28.3 | 24.28 | 392 | 6.7 | 271 | 2 | 14.30 | 12.4 | 1.150 | 1.84 | 2.83 | 5.64 |
| Patient 46 | 51.3 | 32.5 | 2.13 | 1.578 | 814 | 13 | 62.62 | 389 | 6.13 | 360 | 0.96 | 17.70 | 10.1 | 1.752 | 1.76 | 2.46 | 2.64 |
| Patient 47 | 78 | 35.2 | 0.95 | 2.216 | 1016 | 12 | 84.67 | 456 | 6.6 | 326 | 1.41 | 17.00 | 10.7 | 1.589 | 2.39 | 3.04 | 4.00 |
| Patient 48 | 157 | 34.3 | 1.12 | 4.580 | 916 | 21.2 | 43.21 | 462 | 7.01 | 348 | 1.88 | 17.60 | 15.5 | 1.140 | 2.5 | 3.89 | 5.09 |
| Patient 49 | 64.5 | 33.6 | 1.08 | 1.920 | 838.6 | 18.7 | 44.84 | 361 | 5.47 | 271 | 1.6 | 19.40 | 11.7 | 1.660 | 1.6 | 2.69 | 4.82 |
| Patient 50 | 62 | 33 | 2.63 | 1.770 | 849 | 10.5 | 80.86 | 381 | 5.91 | 279 | 1.08 | 17.00 | 11.4 | 1.490 | 3.27 | 2.81 | 5.27 |
| Patient 51 | 86 | 31.3 | 2.1 | 2.750 | 884 | 20.5 | 43.12 | 571 | 5.47 | 75 | 1.4 | 16.20 | 11 | 1.470 | 2.36 | 2.65 | 7.64 |
| Patient 52 | 89.5 | 33.5 | 0.69 | 2.670 | 872.8 | 19.1 | 45.70 | 440 | 5.18 | 386 | 2.02 | 18.20 | 12.7 | 1.430 | 1.549 | 2.46 | 4.82 |
| Patient 53 | 78.2 | 39.3 | 2.38 | 1.990 | 936 | 21.7 | 43.13 | 405 | 6.09 | 373 | 125 | 16.20 | 13 | 1.250 | 1.95 | 3.21 | 5.73 |
| Patient 54 | 12 | 21.1 | 0.93 | 0.570 | 959 | 24.5 | 39.14 | 373 | 6.31 | 306 | 1.04 | 15.40 | 13.4 | 1.150 | 1.4 | 2.02 | 3.18 |
| Patient 55 | 52.4 | 22.9 | 1.93 | 2.300 | 743 | 13.5 | 55.04 | 389 | 6.35 | 400 | 1.3 | 12.80 | 12 | 1.070 | 2.66 | 3.14 | 5.18 |
| Patient 56 | 50.4 | 34.4 | 2.71 | 1.470 | 781 | 14.5 | 53.86 | 415 | 3.7 | 318 | 1.63 | 21.40 | 12.4 | 1.730 | 2.51 | 3.24 | 6.27 |

(suite)

| Patient | Vit C | Alpha Toco | Gamma tocoph | Vit C/E | GSH | GSSG | Ratio G/G | GPX | Prot Thiol | Acide Urique | Sel | Cu | Zn | Cu/Zn | MDA | Tbars | Oxyscale |
|---|---|---|---|---|---|---|---|---|---|---|---|---|---|---|---|---|---|
| Patient 57 | 52.6 | 22.9 | 1.95 | 2.300 | 743 | 13.5 | 55.04 | 389 | 6.35 | 400 | 1.3 | 12.80 | 12 | 1.070 | 2.66 | 3.14 | 5.18 |
| Patient 58 | 68.5 | 26.9 | 2.49 | 2.546 | 1038 | 6 | 173.00 | 470 | 5.7 | 386.8 | 0.97 | 22.20 | 10.9 | 2.037 | 1.83 | 3 | 3.18 |
| Patient 59 | 74 | 17.4 | 1.09 | 0.000 | 1108 | 26 | 42.62 | 703 | 5.96 | 328 | 2.1 | 13.50 | 12 | 1.130 | 0.95 | 2.16 | 4.45 |
| Patient 60 | 47.1 | 29.3 | 2.33 | 1.610 | 797 | 19.6 | 40.66 | 363 | 6.77 | 321 | 1.75 | 17.40 | 12.6 | 1.380 | 2.16 | 2.87 | 5.82 |
| Patient 61 | 58.4 | 25.5 | 1.58 | 2.290 | 882 | 19 | 46.42 | 447 | 7.05 | 424 | 1.28 | 13.50 | 12.8 | 1.050 | 3.2 | 4.02 | 4.73 |
| Patient 62 | 112 | 18.2 | 0.24 | 6.150 | 1558 | 16.3 | 95.58 | 260 | 6.69 | 243.9 | 1.46 | 16.00 | 13.2 | 1.210 | 1.44 | 2.53 | 3.55 |
| Patient 63 | 68.5 | 27.1 | 0.81 | 2.528 | 904 | 8 | 113.00 | 470 | 6.07 | 270 | 1.65 | 16.40 | 11.9 | 1.378 | 2.89 | 3.16 | 4.36 |
| Patient 64 | 81 | 38.4 | 2 | 2.110 | 937 | 19.5 | 48.05 | 367 | 5.8 | 285 | 1.11 | 19.40 | 13.7 | 1.420 | 2.63 | 3.79 | 6.55 |
| Patient 65 | 62.7 | 27.6 | 1.36 | 2.270 | 1044 | 8 | 130.50 | 278 | 5.26 | 335 | 0.74 | 16.40 | 9.4 | 1.740 | 1.79 | 2.93 | 4.09 |
| Patient 66 | 62.7 | 33.6 | 1.27 | 1.870 | 813 | 18.5 | 43.95 | 519 | 6.78 | 280 | 1.56 | 19.80 | 15.4 | 1.290 | 3.05 | 3.08 | 5.18 |
| Patient 67 | 76.9 | 34.5 | 1.08 | 2.230 | 944 | 18 | 32.44 | 488 | 623 | 190 | 1.49 | 18.00 | 11.7 | 1.540 | 2.19 | 2.82 | 5.27 |
| Patient 68 | 61.2 | 33.7 | 1.23 | 1.820 | 836 | 37 | 22.59 | 422 | 6.35 | 404 | 2.1 | 16.20 | 12.6 | 1.290 | 2.34 | 2.98 | 6.18 |
| Patient 69 | 20.7 | 23.6 | 1.59 | 0.880 | 980 | 13.2 | 74.24 | 332 | 5.49 | 399 | 1.11 | 18.40 | 11.2 | 1.640 | 1.64 | 2.88 | 5.18 |
| Patient 70 | 61.2 | 16.8 | 1.07 | 3.643 | 974 | 18 | 54.11 | 339 | 6.36 | 377 | 0.98 | 11.00 | 12 | 0.917 | 1.74 | 2.26 | 3.09 |

EP 2 017 623 B1

(suite)

| Patient | Vit C | Alpha Toco | Gamma tocoph | Vit C/E | GSH | GSSG | Ratio G/G | GPX | Prot Thiol | Acide Urique | Sel | Cu | Zn | Cu/Zn | MDA | Tbars | Oxyscale |
|---|---|---|---|---|---|---|---|---|---|---|---|---|---|---|---|---|---|
| Patient 71 | 64.8 | 25.9 | 0.82 | 2.502 | 794 | 28 | 28.36 | 549 | 6.15 | 219 | 1.31 | 21.00 | 11.5 | 1.826 | 2.05 | 2.43 | 6.36 |
| Patient 72 | 67.5 | 26.2 | 1.13 | 2.580 | 722 | 19 | 38.00 | 457 | 5.07 | 404 | 1.37 | 20.50 | 11.1 | 1.850 | 2 | 2.96 | 5.91 |
| Patient 73 | 75.4 | 30.9 | 1.61 | 2.440 | 660 | 33.7 | 19.58 | 352 | 6.1 | 299 | 1.78 | 14.80 | 13.1 | 1.130 | 1.33 | 2.62 | 5.18 |
| Patient 74 | 98.2 | 24.5 | 0.68 | 4.010 | 818 | 20.3 | 40.30 | 417 | 5.21 | 318 | 1.29 | 16.00 | 14.8 | 1.080 | 1.47 | 2.73 | 4.73 |
| Patient 75 | 75.5 | 22.5 | 0.77 | 3.360 | 847 | 24.3 | 34.86 | 436 | 5.11 | 271 | 1.31 | 17.20 | 12.6 | 1.370 | 1.72 | 2.84 | 5.27 |
| Patient 76 | 18.9 | 26.7 | 1.58 | 0.708 | 982 | 19 | 51.68 | 503 | 6.01 | 456 | 1.23 | 9.80 | 11.9 | 0.824 | 2.42 | 2.4 | 5.18 |
| Patient 77 | 37.5 | 22.4 | 0.87 | 1.670 | 701 | 19.5 | 35.95 | 405 | 5.41 | 315 | 1.34 | 15.40 | 11.5 | 1.340 | 1.83 | 2.64 | 6.45 |
| Patient 78 | 55.1 | 36.7 | 1.14 | 1.500 | 762 | 10.9 | 69.91 | 466 | 5.36 | 188 | 1.21 | 24.00 | 15.4 | 1.560 | 2.54 | 3.35 | 6.18 |
| Patient 79 | 77 | 31.7 | 1.6 | 2.430 | 861 | 16.1 | 53.48 | 439 | 6.6 | 224 | 1.05 | 14.80 | 13.1 | 1.130 | 1.47 | 2.8 | 2.82 |
| Patient 80 | 57.6 | 22.7 | 0.83 | 2.540 | 792 | 23.7 | 33.42 | 363 | 5.87 | 215 | 1.1 | 16.10 | 12.7 | 1.290 | 1.52 | 2.55 | 5.18 |
| Patient 81 | 57 | 22 | 0 | 0.000 | 792 | 23 | 0.00 | 363 | 5 | 215 | 1 | 16.00 | 12 | 0.000 | 1 | 2 | 4.73 |
| Patient 82 | 72.3 | 33 | 2.07 | 2.191 | 1023 | 13.5 | 75.78 | 413 | 6.84 | 235 | 1.27 | 18.40 | 9 | 2.044 | 1.86 | 2.62 | 4.00 |
| Patient 83 | 60.8 | 34.6 | 1.76 | 1.757 | 802 | 4 | 200.50 | 474 | 6.38 | 411 | 1.45 | 17.40 | 10.8 | 1.611 | 1.98 | 2.64 | 2.64 |
| Patient 84 | 101 | 25.4 | 0.9 | 3.980 | 1031 | 31 | 33.26 | 482 | 6.4 | 138 | 1.69 | 15.40 | 11.8 | 1.310 | 0.92 | 1.95 | 4.36 |

(suite)

| Patient | Vit C | Alpha Toco | Gamma tocoph | Vit C/E | GSH | GSSG | Ratio G/G | GPX | Prot Thiol | Acide Urique | Sel | Cu | Zn | Cu/Zn | MDA | Tbars | Oxyscale |
|---|---|---|---|---|---|---|---|---|---|---|---|---|---|---|---|---|---|
| Patient 85 | 68.2 | 232 | 131 | 2.710 | 880 | 10 | 88.00 | 309 | 6.8 | 198 | 0.9 | 18.80 | 11.6 | 1.620 | 137 | 2.36 | 1.73 |
| Patient 86 | 69.3 | 29.5 | 2.26 | 2.350 | 754 | 15.8 | 47.72 | 470 | 6.07 | 251 | 1.3 | 18.20 | 10.4 | 1.750 | 1.7 | 2.99 | 5.36 |
| Patient 87 | 62.2 | 25.2 | 3.3 | 2.470 | 991 | 8.7 | 113.91 | 512 | 6.7 | 241 | 1.23 | 17.00 | 12 | 1.420 | 2.35 | 2.9 | 3.91 |
| Patient 88 | 83.3 | 29.6 | 1.69 | 2.810 | 996 | 42 | 23.71 | 509 | 6.56 | 234 | 1.63 | 21.50 | 14.8 | 1.450 | 1.73 | 2.87 | 5.36 |
| Patient 89 | 70.2 | 30.5 | 1.04 | 2.300 | 1077 | 1.53 | 34.19 | 508 | 5.88 | 409 | 1.83 | 2.30 | 13.3 | 1.530 | 2.42 | 3.56 | 5.36 |
| Patient 90 | 72.6 | 33.5 | 1.94 | 2.170 | 702 | 10.5 | 66.86 | 522 | 5.81 | 475 | 1.89 | 16.20 | 8.4 | 1.930 | 4.16 | 3.99 | 5.45 |
| Patient 91 | 64 | 31.1 | 4.09 | 2.060 | 723 | 12.2 | 59.26 | 411 | 6.4 | 167 | 1.57 | 18.40 | 11.9 | 1.550 | 2.14 | 3.03 | 5.37 |
| Patient 92 | 48 | 25.4 | 0.66 | 1.890 | 824 | 15 | 54.93 | 296 | 5.15 | 327.2 | 0.82 | 14.50 | 12.4 | 1.170 | 1.79 | 2.8 | 3.91 |
| Patient 93 | 58.5 | 22.4 | 2.25 | 2.612 | 891 | 19.5 | 45.69 | 375 | 6.01 | 547.4 | 1.15 | 16.20 | 12.3 | 1.317 | 2.26 | 3.25 | 5.64 |
| Patient 94 | 71.3 | 28.5 | 2.72 | 2.502 | 642 | 39 | 16.46 | 393 | 5.81 | 307 | 1.14 | 18.40 | 12.3 | 1.496 | 2.43 | 3.68 | 7.45 |
| Patient 95 | 99 | 33 | 1.23 | 3.000 | 948 | 26.5 | 35.77 | 500 | 6.17 | 187 | 2.28 | 21.80 | 10 | 2.180 | 3.88 | 4.45 | 6.82 |
| Patient 96 | 66.5 | 23.4 | 1.06 | 2.840 | 621 | 113 | 5.50 | 465 | 6.37 | 282 | 1.38 | 16.20 | 14.4 | 1.130 | 2.1 | 3.09 | 6.55 |
| Patient 97 | 155 | 31.6 | 2.133 | 4.910 | 951 | 16.3 | 58.34 | 427 | 5.74 | 148.8 | 1.08 | 20.60 | 12.9 | 1.600 | 2.01 | 3.16 | 6.18 |
| Patient 98 | 166 | 25.6 | 0.64 | 6.480 | 954 | 22.2 | 42.97 | 275 | 5.97 | 327.2 | 1.25 | 13.60 | 11.8 | 1.150 | 1.52 | 2.61 | 4.27 |

(suite)

| Patient | Vit C | Alpha Toco | Gamma tocoph | Vit C/E | GSH | GSSG | Ratio G/G | GPX | Prot Thiol | Acide Urique | Sel | Cu | Zn | Cu/Zn | MDA | Tbars | Oxyscale |
|---|---|---|---|---|---|---|---|---|---|---|---|---|---|---|---|---|---|
| Patient 99 | 79.2 | 31.7 | 0.74 | 2.500 | 776 | 15 | 51.73 | 581 | 5.23 | 285.6 | 1.48 | 21.80 | 14.4 | 1.510 | 2.04 | 2.52 | 5.82 |
| Patient 100 | 73 | 35.4 | 1.09 | 2.060 | 769 | 16.5 | 46.61 | 397 | 5.6 | 214 | 1.68 | 13.80 | 12.9 | 1.070 | 2.2 | 3.82 | 6.55 |
| Patient 101 | 89.9 | 23.3 | 0.63 | 3.860 | 707 | 41.7 | 16.95 | 523 | 5.46 | 250 | 2.18 | 22.40 | 12.1 | 1.850 | 2.86 | 3.65 | 8.09 |
| Patient 102 | 70.5 | 35.5 | 1.33 | 1.990 | 715 | 57.9 | 12.35 | 380 | 5.77 | 272 | 1.51 | 17.20 | 13.5 | 1.270 | 3.96 | 4.3 | 7.55 |
| Patient 103 | 59 | 26.4 | 0.9 | 2135 | 881 | 12 | 73.42 | 371 | 6.14 | 319 | 1.26 | 17.60 | 8.5 | 2.071 | 1.9 | 3.11 | 3.64 |
| Patient 104 | 76.6 | 28 | 2.49 | 2.740 | 600 | 22.2 | 27.03 | 600 | 5 | 263 | 1.63 | 28.20 | 12.8 | 2.200 | 1.89 | 3.32 | 8.18 |
| Patient 105 | 69.1 | 26.5 | 0.87 | 2.610 | 1063 | 8.5 | 125.06 | 456 | 6.18 | 181 | 128 | 19.40 | 123 | 1.580 | 1.89 | 2.41 | 3.45 |
| Patient 106 | 73.4 | 28.1 | 2.47 | 2.612 | 1271 | 9.5 | 133.79 | 502 | 6.42 | 198 | 1.58 | 15.00 | 10.3 | 1.456 | 2.4 | 3.06 | 4.91 |
| Patient 107 | 92.8 | 20.2 | 0.43 | 4.590 | 960 | 5 | 192.00 | 317 | 6.12 | 96 | 1.47 | 21.80 | 9.1 | 2.400 | 1.78 | 2.15 | 2.82 |
| Patient 108 | 70.5 | 17.4 | 0.67 | 4.052 | 839 | 15.5 | 54.13 | 356 | 5.07 | 300 | 1.03 | 18.60 | 10.1 | 1.842 | 1.55 | 2.22 | 4.00 |
| Patient 109 | 42.1 | 25.7 | 1.42 | 1.638 | 884 | 38 | 23.26 | 678 | 635 | 342 | 1.37 | 18.40 | 9.8 | 1.878 | 3.07 | 3.97 | 6.36 |
| Patient 110 | 54.8 | 35.8 | 2.58 | 1.531 | 989 | 15.5 | 63.81 | 472 | 639 | 332 | 138 | 13.50 | 11.6 | 1.164 | 2.73 | 2.88 | 4.36 |
| Patient 111 | 61.6 | 27.7 | 0.64 | 2.224 | 1149 | 8 | 143.63 | 429 | 5.78 | 348 | 1.28 | 12.70 | 11 | 1.155 | 2.39 | 2.52 | 4.82 |
| Patient 112 | 77.4 | 26.8 | 1.81 | 2.888 | 1001 | 24.5 | 40.86 | 499 | 629 | 238 | 1.33 | 17.50 | 10.7 | 1.636 | 1.61 | 2.58 | 4.45 |

EP 2 017 623 B1

(suite)

| Patient | Vit C | Alpha Toco | Gamma tocoph | Vit C/E | GSH | GSSG | Ratio G/G | GPX | Prot Thiol | Acide Urique | Sel | Cu | Zn | Cu/Zn | MDA | Tbars | Oxyscale |
|---|---|---|---|---|---|---|---|---|---|---|---|---|---|---|---|---|---|
| Patient 113 | 90.2 | 55 | 1.38 | 1.640 | 817 | 6.5 | 125.69 | 554 | 6.33 | 122 | 1.3 | 17.40 | 10.1 | 1.723 | 1.79 | 2.54 | 3.18 |
| Patient 114 | 12 | 29.7 | 1.71 | 0.400 | 1046 | 10.1 | 103.56 | 402 | 5.7 | 257 | 1.24 | 15.40 | 13.9 | 1.110 | 1.77 | 1.52 | 3.27 |
| Patient 115 | 83 | 25.4 | 2.53 | 3.270 | 866.6 | 12.2 | 71.03 | 323 | 5.75 | 217 | 1.15 | 18.20 | 11.1 | 1.640 | 1.862 | 2.92 | 4.36 |
| Patient 116 | 77.6 | 47.5 | 1.05 | 1.640 | 836 | 13.2 | 63.33 | 368 | 6.06 | 178.5 | 1.56 | 18.00 | 13.9 | 1.290 | 1.64 | 2.67 | 3.45 |
| Patient 117 | 83.9 | 36.7 | 1.5 | 2.290 | 1082 | 16.6 | 65.18 | 522 | 55 | 327.2 | 1.56 | 13.50 | 11.6 | 1.160 | 2.07 | 2.81 | 4.36 |
| Patient 118 | 52.5 | 38.9 | 3.59 | 1.350 | 803 | 33.5 | 23.97 | 370 | 6.41 | 321 | 1.39 | 18.80 | 11.1 | 1.690 | 2.5 | 2.97 | 6.27 |
| Patient 119 | 69.9 | 29 | 0.75 | 2.410 | 921 | 31.4 | 29.33 | 370 | 5.66 | 424 | 1.24 | 16.00 | 14.5 | 1.100 | 1.57 | 2.78 | 4.73 |
| Patient 120 | 131 | 30.7 | 2.09 | 4.270 | 1037 | 10.2 | 101.67 | 508 | 5.85 | 118 | 1.44 | 14.50 | 14.7 | 0.990 | 1.79 | 2.75 | 2.45 |
| Patient 121 | 51.7 | 30.4 | 1.94 | 1.900 | 895 | 48.3 | 18.53 | 423 | 6.4 | 356 | 1.37 | 17.80 | 12.1 | 1.140 | 1.92 | 2.09 | 2.36 |
| Patient 122 | 64.6 | 28 | 1.22 | 2.310 | 826 | 22 | 17.55 | 400 | 6.65 | 208 | 1.4 | 16.20 | 13 | 1.250 | 2.33 | 2.64 | 5.73 |
| Patient 123 | 72 | 25.1 | 1.92 | 2.869 | 585 | 22.5 | 26.00 | 630 | 5.59 | 330 | 1.19 | 20.00 | 8.9 | 2.247 | 2.73 | 3.63 | 7.18 |
| Patient 124 | 48 | 31.5 | 2.01 | 1.520 | 1010 | 21 | 48.10 | 364 | 6.4 | 328 | 0.89 | 18.60 | 13.3 | 1.400 | 1.42 | 2.57 | 4.00 |
| Patient 125 | 59.3 | 28 | 1.28 | 2.120 | 920 | 12.3 | 74.80 | 434 | 5.61 | 291.5 | 0.96 | 18.30 | 10.5 | 1.740 | 1.65 | 3.08 | 4.45 |
| Patient 126 | 71.9 | 24.7 | 1.82 | 2.911 | 834 | 18 | 46.33 | 428 | 6.25 | 295 | 1.13 | 22.40 | 9.1 | 2.462 | 1.95 | 3.29 | 5.91 |

EP 2 017 623 B1

(suite)

| Patient | Vit C | Alpha Toco | Gamma tocoph | Vit C/E | GSH | GSSG | Ratio G/G | GPX | Prot Thiol | Acide Urique | Sel | Cu | Zn | Cu/Zn | MDA | Tbars | Oxyscale |
|---|---|---|---|---|---|---|---|---|---|---|---|---|---|---|---|---|---|
| Patient 127 | 69 | 26 | 2.27 | 2.650 | 1127 | 18.9 | 59.63 | 468 | 6.7 | 113 | 1.41 | 15.60 | 12 | 1.300 | 1.68 | 2.26 | 3.91 |
| Patient 128 | 178 | 28 | 0.62 | 6.360 | 679 | 16.3 | 41.66 | 440 | 5.42 | 247 | 1.35 | 18.20 | 11.8 | 1.540 | 1.53 | 2.78 | 5.27 |
| Patient 129 | 51.7 | 21.5 | 1.41 | 2.400 | 1050 | 18.7 | 56.13 | 395 | 6.37 | 359 | 1.25 | 15.00 | 12.8 | 1.170 | 1.41 | 2.68 | 2.91 |
| Patient 130 | 77.3 | 38.2 | 1.39 | 2.020 | 1025 | 15.8 | 64.87 | 328 | 5.33 | 314 | 1.33 | 16.50 | 12.9 | 1.280 | 1.68 | 3.13 | 4.82 |
| Patient 131 | 34.2 | 28.3 | 2.08 | 1.208 | 1123 | 14 | 83.19 | 423 | 5.34 | 393 | 1.05 | 17.60 | 12.60 | 1.397 | 2 | 3.06 | 5.45 |
| Patient 132 | 122 | 30.1 | 23 | 4.050 | 1033 | 16 | 64.56 | 629 | 7.1 | 222 | 1.52 | 17.40 | 18.70 | 0.930 | 2 | 3 | 3.73 |
| Patient 133 | 78.7 | 39.4 | 2.25 | 2.000 | 862 | 18 | 47.10 | 590 | 5.83 | 227 | 1.37 | 19.50 | 13.70 | 1.420 | 2 | 3.34 | 6.18 |
| Patient 134 | 84 | 21.7 | 1 | 3.870 | 936 | 75 | 12.48 | 459 | 5.13 | 318 | 1.02 | 12.50 | 12.9 | 0.970 | 2.43 | 3.3 | 6.91 |
| Patient 135 | 64.2 | 28.5 | 1.21 | 2.250 | 863 | 16.6 | 51.99 | 382 | 5.9 | 269 | 1.1 | 23.50 | 14.1 | 1.670 | 2.05 | 2.84 | 4.82 |
| Patient 136 | 51 | 29.5 | 0.77 | 1.730 | 1058 | 16 | 66.13 | 399 | 6 | 301 | 1.19 | 12.80 | 9.5 | 1.350 | 1.82 | 2.95 | 3.91 |
| Patient 137 | 66 | 21.3 | 2.05 | 3.100 | 1098 | 8.4 | 130.71 | 341 | 5.48 | 460 | 1.05 | 17.20 | 13.7 | 1.100 | 1.96 | 2.87 | 3.64 |
| Patient 138 | 78.1 | 39.7 | 2.98 | 1.970 | 930 | 12.9 | 72.09 | 410 | 6.68 | 325 | 15 | 15.20 | 11.1 | 1.370 | 2.02 | 2.89 | 3.09 |
| Patient 139 | 58.7 | 33.3 | 1.6 | 1.760 | 1003 | 13.5 | 74.30 | 422 | 6.21 | 294.5 | 1.49 | 17.20 | 10.2 | 1.690 | 1.77 | 2.39 | 3.55 |
| patient 140 | 20 | 35 | 2 | 0.571 | 1300 | 20 | 65.00 | 500 | 6 | 320 | 0.6 | 17.00 | 8 | 2.125 | 2.4 | 3.04 | 6.36 |

EP 2 017 623 B1

(suite)

| Patient | Vit C | Alpha Toco | Gamma tocoph | Vit C/E | GSH | GSSG | Ratio G/G | GPX | Prot Thiol | Acide Urique | Sel | Cu | Zn | Cu/Zn | MDA | Tbars | Oxyscale |
|---|---|---|---|---|---|---|---|---|---|---|---|---|---|---|---|---|---|
| Patient 141 | 15.3 | 33.9 | 0.69 | 0.450 | 901 | 11 | 82.66 | 408 | 5.9 | 323 | 1.5 | 17.80 | 12.10 | 1.470 | 1 | 3.12 | 4.27 |
| Patient 142 | 67.4 | 32.3 | 1.31 | 2.090 | 975 | 33 | 29.91 | 470 | 6.4 | 375 | 1.58 | 16.80 | 12.80 | 1.310 | 2 | 2.79 | 5.27 |
| Patient 143 | 79.2 | 39.6 | 1.63 | 2.000 | 1007 | 6.5 | 154.92 | 567 | 6.07 | 306 | 2.04 | 15.00 | 12.6 | 1.190 | 2.35 | 3.06 | 3.36 |
| Patient 144 | 57 | 19.9 | 0.99 | 2.864 | 924 | 13 | 71.08 | 461 | 6.19 | 293 | 1.39 | 18.20 | 9.8 | 1.837 | 1.6 | 2.44 | 3.18 |
| Patient 145 | 66.8 | 18.2 | 1.09 | 3.670 | 995 | 26.1 | 38.12 | 405 | 6.03 | 263 | 1.21 | 14.60 | 11 | 1.330 | 1.72 | 3.14 | 5.91 |
| Patient 146 | 61.2 | 22 | 1.49 | 2.780 | 894 | 19 | 47.05 | 447 | 6.94 | 267 | 1.39 | 13.00 | 11.3 | 1.330 | 1.98 | 2.51 | 4.45 |
| Patient 147 | 53.9 | 22.6 | 0.57 | 2.385 | 861 | 14.5 | 59.38 | 579 | 6.08 | 318 | 1.51 | 20.00 | 12.8 | 1.563 | 1.47 | 2.34 | 2.09 |
| Patient 148 | 62 | 31.8 | 1.84 | 1.950 | 975 | 25.6 | 38.09 | 347 | 6.4 | 273 | 1.24 | 20.30 | 13.6 | 1.490 | 1.74 | 2.87 | 5.82 |
| Patient 149 | 78.1 | 44.8 | 1.17 | 1.740 | 1110 | 18 | 61.33 | 471 | 5.01 | 279.6 | 1.74 | 18.00 | 12.10 | 1.490 | 2 | 2.89 | 5.73 |
| Patient 150 | 65.9 | 25.7 | 1.52 | 2.564 | 1040 | 40 | 26.00 | 495 | 5.72 | 305 | 126 | 19.90 | 12.60 | 1.579 | 2 | 3.16 | 6.64 |
| Patient 151 | 95.4 | 23.7 | 2 | 4.025 | 972 | 24 | 40.50 | 430 | 6.19 | 460 | 1.5 | 17.00 | 13.50 | 1.259 | 2 | 3.29 | 5.73 |
| Patient 152 | 49 | 43.8 | 1.36 | 1.120 | 918 | 16 | 57.38 | 397 | 3.52 | 291.5 | 2.08 | 24.20 | 11.00 | 2.200 | 2 | 3.18 | 6.09 |
| Patient 153 | 79 | 23.6 | 1.49 | 3.350 | 1163 | 17 | 69.23 | 419 | 5.97 | 309 | 1.22 | 17.10 | 11.00 | 1.370 | 3 | 3.17 | 5.73 |
| Patient 154 | 81.8 | 27.3 | 1.6 | 3.000 | 853 | 21 | 40.62 | 478 | 6.66 | 270 | 1.06 | 20.40 | 12.40 | 1.630 | 2 | 3.22 | 5.73 |

(suite)

| Patient | Vit C | Alpha Toco | Gamma tocoph | Vit C/E | GSH | GSSG | Ratio G/G | GPX | Prot Thiol | Acide Urique | Sel | Cu | Zn | Cu/Zn | MDA | Tbars | Oxyscale |
|---|---|---|---|---|---|---|---|---|---|---|---|---|---|---|---|---|---|
| Patient 155 | 92.7 | 27.9 | 1.46 | 3.320 | 886 | 16 | 54.69 | 362 | 6 | 332 | 0.89 | I7.00 | 11.60 | 1.470 | 2 | 2.86 | 3.09 |
| Patient 156 | 15.3 | 42 | 1.58 | 0.360 | 916.4 | 10 | 88.97 | 492 | 5.6 | 166 | 1.05 | 18.60 | 12.10 | 1.540 | 2 | 2.9 | 4.64 |
| Patient 157 | 88.7 | 24.1 | 1.41 | 3.680 | 1314 | 13 | 101.86 | 319 | 5.76 | 242 | 1.09 | 19.80 | 11.00 | 1.800 | 1 | 2.57 | 3.45 |
| Patient 158 | 46.9 | 25.7 | 1.2 | 1.825 | 1010 | 10 | 101.00 | 460 | 5.54 | 216 | 1.13 | 20.00 | 9.50 | 2.105 | 2 | 3.4 | 4.91 |
| Patient 159 | 66.9 | 26.5 | 1.71 | 2.525 | 1265 | 18 | 72.29 | 411 | 5.96 | 351 | 1.18 | 18.40 | 8.20 | 2.244 | 2 | 3.63 | 5.82 |
| Patient 160 | 60 | 20 | 2.5 | 3.000 | 600 | 20 | 30.00 | 460 | 6 | 240 | 1.1 | 20.00 | 10.00 | 2.000 | 3 | 3 | 7.09 |
| Patient 161 | 63 | 30 | 1.67 | 2.100 | 969 | 14 | 68.24 | 497 | 6.4 | 291 | 1.42 | 15.60 | 12.10 | 1.290 | 3 | 3.47 | 5.27 |
| Patient 162 | 52 | 17.2 | 3.2 | 3.020 | 661 | 23 | 28.99 | 569 | 6.6 | 155 | 1.45 | 17.40 | 12.20 | 1.430 | 3 | 3.76 | 6.27 |
| Patient 163 | 82.3 | 31 | 1.34 | 2.650 | 947 | 26 | 36.85 | 336 | 5.81 | 238 | 1.07 | 20.60 | 12.50 | 1.650 | 2 | 3.09 | 6.18 |
| Patient 164 | 42.7 | 26.1 | 1.72 | 1.636 | 1083 | 14 | 80.22 | 439 | 5.82 | 380 | 1.16 | 20.20 | 13.80 | 1.464 | 2 | 2.71 | 4.45 |
| Patient 163 | 101.2 | 35.4 | 1.2 | 2.860 | 880 | 16 | 56.41 | 258 | 5.33 | 234 | 1.84 | 20.00 | 10.70 | 1.870 | 2 | 3.11 | 6.27 |
| Patient 166 | 75 | 31.3 | 1.86 | 2.400 | 834.6 | 20 | 41.32 | 510 | 6.7 | 251 | 1.05 | 17.40 | 12.50 | 1.390 | 2 | 3.02 | 5.64 |
| Patient 167 | 50 | 24.6 | 0.88 | 2.030 | 1589 | 12.2 | 130.25 | 519 | 5.1 | 337 | 2.02 | 15.40 | 9.7 | 1.590 | 1.32 | 2.21 | 2.64 |
| Patient 168 | 126.6 | 26.7 | 1.55 | 4.740 | 867 | 10.1 | 85.84 | 403 | 6.57 | 366 | 1.44 | 16.50 | 12.4 | 1.330 | 2.05 | 3.31 | 3.91 |

(suite)

| Patient | Vit C | Alpha Toco | Gamma tocoph | Vit C/E | GSH | GSSG | Ratio G/G | GPX | Prot Thiol | Acide Urique | Sel | Cu | Zn | Cu/Zn | MDA | Tbars | Oxyscale |
|---|---|---|---|---|---|---|---|---|---|---|---|---|---|---|---|---|---|
| Patient 169 | 12.6 | 41.9 | 1.57 | 1.971 | 899 | 10.5 | 84.67 | 426 | 6.45 | 256 | 1.66 | 15.00 | 9.3 | 1.613 | 3.05 | 3.4 | 4.91 |
| Patient 170 | 13.9 | 28.6 | 0.64 | 2.930 | 1171 | 20.1 | 58.26 | 372 | 6.54 | 278 | 1.03 | 19.20 | 15.9 | 1.210 | 1.93 | 2.72 | 4.64 |

ANNEXE 2

| Abréviation | Désignation | Unité | Normes |
|---|---|---|---|
| Vit C | Vitamine C | μmol/l | 15 - 62 |
| Alpha tocoph | Alpha tocophérol | μmol/l | 18 - 48.5 |
| Gamma tocoph | Gamma tocophérol | μmol/l | 1.4 - 3.6 |
| Vit C/E | Ratio Vitamine C / Vitamine E | | > 1,3 |
| GSH | Glutathion | μmol/l | 729 - 1203 |
| GSSG | Glutathion oxydé | μmol/l | 5.5 - 19.3 |
| Ratio G/G | Ratio Glutathion/Glutathion oxydé | | 40 - 155 |
| GPX | Glutathion Péroxydase | U/l | 300 - 450 |
| Prot Thiol | Protéines Thiols | μmol/g de prot | 5.8 - 7.7 |
| AcideUrique | Acide urique | μmol/l | 145 - 460 |
| Sel | Sélénium | μmol/l | 0.8 - 1.25 |
| Cu | Cuivre | μmol/l | 12.4 - 25.9 |
| Zn | Zinc | μmol/l | 11.7 - 17.3 |
| Cu/Zn | Ratio Cuivre/Zinc | | proche de 1 |
| MDA | Malon Dialdéhyde Plasmatique | μmol/l | 1.12 - 1.81 |
| T-Bars | Taux serique des substances réagissant avec l'acide thio-barbiturique | μmol/l | 2.13 - 2.86 |

**Revendications**

1. Procédé de prédiction d'un score représentatif du stress oxydatif d'un patient sur une échelle de 0 à 10 **caractérisé par**
   la succession des étapes suivantes :

   - on sélectionne un parmi les deux groupes de cinq marqueurs biologiques suivants :

      - le premier groupe étant constitué :du cuivre Cu, du zinc Zn, du rapport glutathion réduit/glutathion oxydé (ratio G/G), des protéines thiols (prot th) et du malon dialdéhyde plasmatique (MDA) et ;
      - le second groupe étant constitué : du cuivre Cu, du zinc Zn, des protéines thiols (prot. th), du malon dialdéhyde plasmatique (MDA) et des T-Bars, c'est-à-dire des substances qui réagissent avec l'acide thio-barbiturique ;

   - on évalue par dosage, en particulier à partir de prélèvements sanguins, les marqueurs biologiques du groupe sélectionné du patient, et
   - on calcule le score prédictif Sp spécifique au patient en utilisant une relation de prédiction basée sur le dosage des cinq marqueurs du groupe selectionné.

2. Procédé selon la revendication 1,
   **caractérisé en ce que**
   la relation de prédiction est la relation suivante :

$$Sp = 6.8127106 + 0.5105519xCu/Zn - 0.026794xRatio\ G/G - 0.671527xprot\ th + 1.5006322xMDA + 0.0001327x(ratio\ G/G-63.8422)x(ratio\ G/G-63.8422) - 0.623101x(MDA-2.03113)x(MDA-2.03113).$$

3. Procédé selon la revendication 1,
   **caractérisé en ce que**
   la relation de prédiction est la relation suivante :

$$Sp' = 3.0818+0.27018xCu/Zn,\ -0.64301xprot\ th + 0.91474xMDA+1.16677xT\text{-}Bars+0.99643x(Cu/Zn-1.52488)x(MDA-2.03113)+0.9099x(Cu/Zn-1.52488)x(T\text{-}Bars-2.91639)+0.29203x(prot\ th-5.9951)x(MDA-2.03113)$$

## Claims

1. Method of predicting a score representative of the oxidative stress of a patient on a scale of 0 to 10
   **characterised by**
   the succession of the following steps:

   - one of the following two groups of five biological markers is selected:

     - the first group consisting of: copper Cu, zinc Zn, the reduced glutathione/oxidized glutathione ratio (ratio G/G), the thiol proteins (prot th) and plasma malon dialdehyde (MDA) and;
     - the second group consisting of: copper Cu, zinc Zn, the thiol proteins (prot th), plasma malon dialdehyde (MDA) and the T-Bars, i.e. the substances which react with thiobarbituric acid;

   - the biological markers of the selected group of the patient are evaluated by assaying, in particular on the basis of blood samples, and
   - the predictive score Sp specific to the patient is calculated using a prediction relationship based on the assaying of the five markers of the selected group.

2. Method as claimed in claim 1,
   **characterised in that**
   the prediction relationship is the following relationship:

$$Sp = 6.8127106 + 0.5105519xCu/Zn - 0.026794xRatio\ G/G - 0.671527xprot\ th + 1.5006322xMDA + 0.0001327x(ratio\ G/G-63.8422)x(ratio\ G/G-63.8422) - 0.623101x(MDA-2.03113)x(MDA-2.03113).$$

3. Method as claimed in claim 1,
   **characterised in that**
   the prediction relationship is the following relationship:

$$Sp' = 3.0818+0.27018xCu/Zn,\ -0.64301xprot\ th + 0.91474xMDA+1.16677xT\text{-}Bars+0.99643x(Cu/Zn-1.52488)x(MDA-2.03113)+0.9099x(Cu/Zn-1.52488)x(T\text{-}Bars-2.91639)+0.29203x(prot\ th-5.9951)x(MDA-2.03113).$$

**Patentansprüche**

1. Verfahren zur Vorhersage eines repräsentativen Scores des oxidativen Stresses eines Patienten auf einer Skala von 0 bis 10,
**gekennzeichnet durch**
die Aufeinanderfolge der folgenden Schritte:

 - Es wird eine der folgenden zwei Gruppen von fünf biologischen Markern ausgewählt:

  - die erste Gruppe, bestehend aus: Kupfer Cu, Zink Zn, dem Verhältnis reduziertes Glutathion/oxidiertes Glutathion (Verhältnis G/G), den Proteinthiolen (prot th) und plasmatischem Malondialdehyd (MDA); oder
  - die zweite Gruppe, bestehend aus: Kupfer Cu, Zink Zn, den Proteinthiolen (prot th), plasmatischem Malondialdehyd (MDA) und den TBARS, das heißt den Substanzen, welche mit Thiobarbitursäure reagieren;

  - es werden **durch** quantitative Bestimmung, insbesondere ausgehend von entnommenen Blutproben, die biologischen Marker der ausgewählten Gruppe des Patienten ausgewertet, und
  - es wird der für den Patienten spezifische Vorhersage-Score Sp unter Verwendung einer Vorhersagebeziehung berechnet, die auf der quantitativen Bestimmung der fünf Marker der ausgewählten Gruppe beruht.

2. Verfahren nach Anspruch 1,
**dadurch gekennzeichnet, dass**
die Vorhersagebeziehung die folgende Beziehung ist:

```
Sp    =    6,8127108    +    0,5105519xCu/Zn    -
0,026794xVerhältnis  G/G  -  0,671527xprot  th  +
```

```
1,5006322xMDA    +    0,0001327x(Verhältnis    G/G-
63,8422)x(Verhältnis G/G-63,8422) - 0,623101x(MDA-
2,03113)x(MDA-2,03113).
```

3. Verfahren nach Anspruch 1,
**dadurch gekennzeichnet, dass**
die Vorhersagebeziehung die folgende Beziehung ist:

```
Sp' = 3,0818 + 0,27018xCu/Zn - 0,64301xprot th +
0,91474xMDA    +    1,16677xTBARS    +    0,99643x(Cu/Zn-
1,52488)x(MDA-2,03113)        +        0,9099x(Cu/Zn-
1,52488)x(TBARS-2,91639)    +    0,29203x(prot    th-
5,9951)x(MDA-2,03113).
```

## FIGURE

Première variante

Seconde variante

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1793230 A